## Europäisches Patentamt

## European Patent Office

## Office européen des brevets

(11) Veröffentlichungsnummer: **0 235 117**
**A1**

# (12) EUROPÄISCHE PATENTANMELDUNG

(21) Anmeldenummer: **87890017.4**

(22) Anmeldetag: **29.01.87**

(51) Int. Cl.⁴: **B 01 D 11/04**
C 12 P 7/06, C 07 C 27/34,
C 07 C 29/86, C 12 P 7/16,
B 01 D 11/00

(30) Priorität: **06.02.86 AT 289/86**

(43) Veröffentlichungstag der Anmeldung:
**02.09.87 Patentblatt 87/36**

(84) Benannte Vertragsstaaten:
**DE ES FR GB IT NL**

(71) Anmelder: **Vogelbusch Gesellschaft m.b.H.**
**Blechturmgasse 11**
**A-1050 Wien (AT)**

(72) Erfinder: **Schmidt, Alfred, Prof.Dipl.-Ing.**
**Pacassistrasse 29**
**A-1130 Wien (AT)**

**Windsperger, Alfred, Dipl.-Ing.Dr.**
**Reismannhof 14/23**
**A-1120 Wien (AT)**

**Friedl, Anton, Dipl.-Ing.**
**Münzwardeingasse 8/21**
**A-1060 Wien (AT)**

(74) Vertreter: **Wolfram, Gustav, Dipl.-Ing.**
**Schwindgasse 7 P.O. Box 205**
**A-1041 Wien (AT)**

(54) **Verfahren zur Abtrennung von sauerstoffhältigen organischen Verbindungen aus wässerigen Medien.**

(57) Die Abtrennung erfolgt durch flüssig-flüssig-Extraktion mit einem organischen Extraktionsmittel.

Um in nur einem Extraktionsschritt eine ausreichende und selektive Abtrennung der organischen Verbindungen zu ermöglichen, ohne daß nennenswerte Mengen an Extraktionsmittel im wässerigen Raffinat gelöst bleiben und ohne daß gegebenenfalls in den wässerigen Medien vorhandene Mikroorganismenkulturen geschädigt werden, wird ein Extraktionsmittel, welches einen Gehalt von bis zu 100 Massen% an wenigstens einem Aldehyd mit 4 bis 20 Kohlenstoffatomen aufweist, eingesetzt.

## Beschreibung

Verfahren zur Abtrennung von sauerstoffhältigen organischen Verbindungen aus wässerigen Medien

Die Erfindung betrifft ein Verfahren zur Abtrennung von sauerstoffhältigen organischen Verbindungen, wie Alkoholen, Aldehyden, Ketonen, Äthern, höheren Carbonsäuren und Estern, aus wässerigen Medien durch flüssig-flüssig-Extraktion mit einem organischen Extraktionsmittel.

An sauerstoffhältigen, organischen Verbindungen, welche erfindungsgemäß aus wässerigen Medien abgetrennt werden können, sind beispielsweise Äthanol, Amylalkohole, Butanol, Aceton und Furfurol zu nennen.

Trennt man solche Verbindungen destillativ ab, muß mit hohem Energieverbrauch gerechnet werden. Zudem muß - beispielsweise bei der Gewinnung von Fermentationsprodukten aus Fermentationsmaischen - die Temperaturbelastung bei der Destillation in Betracht gezogen werden, welche sich auch durch eine aufwendige Vakuumdestillation nicht immer ganz vermeiden läßt. Eine Erhitzung von Maischen auf höhere Temperaturen verhindert aber die Rückführung der von Fermentationsprodukten weitgehend befreiten Maischen in die Fermentationsstufe.

Die Abtrennung der eingangs erwähnten Verbindungen ist aber gerade bei der Gewinnung der Produkte von Fermentations-(Gär-)prozessen aus wässerigen Gärmedien von größter Bedeutung:

Üblicherweise wurden bisher beispielsweise bei der großtechnischen Herstellung von Äthanol durch alkoholische Gärung relativ verdünnte Maischen zur Fermentation eingesetzt, da das Fermentationsprodukt Äthanol schon ab Konzentrationen von etwa 5 % toxisch auf die zur Fermentation eingesetzten Mikroorganismen wirkt, was zu einer Gärhemmung führt. Der Fermentationsprozeß wird meist diskontinuierlich durchgeführt und die Aufarbeitung der vergorenen Maische erfolgt mittels Destillation. Infolge der erforderlichen hohen Maischeverdünnung müssen große Wassermengen durch die einzelnen Verfahrensstufen geführt werden, womit ein entsprechender Energieaufwand verbunden ist.

Durch Verbesserungen bei der Rohstoffaufbereitung und bei der Destillation kann der Energiebedarf nur mehr geringfügig gesenkt werden.

Man ist daher bestrebt, eine weitere Produktivitätssteigerung und Energiebedarfssenkung durch den Einsatz konzentrierterer Maischen bzw. Fermentationsmedien sowie durch kontinuierliche Fermentationsführung zu erzielen. Konzentrierte Maischen können aber nur vergoren werden, wenn die Konzentration der Gärprodukte - wie Äthanol - während der Fermentation unter der Hemmschwelle gehalten wird.

Zur Abtrennung des Produktes aus der Fermentationsmaische während der Fermentation ist grundsätzlich die direkte Entfernung aus dem Fermenter, wie z.B. durch Vakuumfermentation oder durch $CO_2$-Strippen, oder aber die Abtrennung mittels Zirkulieren der Maische durch ein externes Abtrennsystem möglich.

Die Durchführung der Vakuumfermentation bringt insbesondere bei technischen Anlagen Probleme bei der Gärführung, weiters ist die anschließende Kompression der gebildeten Kohlensäure unökonomisch.

Die Abtrennung des Gärproduktes durch Strippen mit $CO_2$ wurde bislang vorwiegend im Labormaßstab erprobt, bei den erforderlichen Abtrennleistungen kann es bereits zu Störungen der Gärung kommen.

Als externes Abtrennsystem können Adsorption, Extraktion, Membranverfahren oder Verfahren mit Nutzung der flüssig-dampf-Anreicherung eingesetzt werden.

Als Extraktionsmittel bei der flüssig-flüssig-Extraktion wurden bereits die meisten gängigen Lösungsmittel untersucht. Allerdings weisen die in Frage kommenden Lösungsmittel relativ hohe Löslichkeit in Wasser bzw. in dem wässerigen Fermentationsmedium auf und wirken zumeist stark toxisch auf die bei der Fermentation verwendeten Mikroorganismen.

Die gleichen Überlegungen bezüglich der Substratkonzentration in Fermentationsflüssigkeiten bzw. bezüglich ökonomischer Fermentationsführung gelten auch für andere Gärungen, beispielsweise für die Butanol-Aceton-Gärung.

In der DE-A-31 12 603 ist ein dreistufiges Verfahren zur Abtrennung niederer aliphatischer Alkohole aus Fermentationsflüssigkeiten beschrieben. Konkret als gewinnbares Gärprodukt erwähnt ist ausschließlich Äthanol.

In der ersten Stufe wird die Fermentationsflüssigkeit vorzugsweise mit einem höheren n-Alkohol extrahiert. Zur Rückgewinnung des Restgehaltes des im ersten Extraktionsschritt eingesetzten Lösungsmittels aus der erhaltenen wässerigen Raffinatphase wird in der zweiten Stufe die Raffinatphase mit einem unpolaren zweiten Lösungsmittel, insbesondere mit einem Alkan mit 5 bis 18 Kohlenstoffatomen, extrahiert. Die beiden nacheinander zur Extraktion eingesetzten Lösungsmittel müssen in einer dritten Stufe wieder voneinander getrennt werden.

Zur Durchführung des bekannten Verfahrens sind zwei Extraktionseinrichtungen und zwei Trennanlagen notwendig. Der energetische und apparative Aufwand ist somit sehr hoch. Dazu kommt noch, daß jeweils bei möglichst hohen Systemtemperaturen bis 60°C extrahiert werden soll.

Die Erfindung stellt sich die Aufgabe, die dargelegten Schwierigkeiten und Nachteile bekannter Abtrennverfahren zu vermeiden und in nur einem Extraktionsschritt eine ausreichende und selektive Abtrennung sauerstoffhältiger organischer Verbindungen aus wässerigen Medien zu ermöglichen, ohne daß nennenswerte Mengen an Extraktionsmittel im wässerigen Raffinat gelöst bleiben und ohne daß gegebenenfalls in den wässerigen Medien vorhandene Mikroorganismenkulturen durch toxische Wirkungen geschädigt werden.

Diese Aufgabe wird bei einem Verfahren der eingangs definierten Art erfindungsgemäß dadurch gelöst, daß ein Extraktionsmittel eingesetzt wird, welches einen Gehalt von bis zu 100 Massen% an wenigstens einem Aldehyd mit 4 bis 20 Kohlenstoffatomen aufweist.

Eine sauerstoffhältige organische Verbindung kann aus wässerigen Medien abgetrennt werden, sofern sie keine chemischen Reaktionen mit den jeweils eingesetzten Aldehyden eingeht.

Der Einsatz des erfindungsgemäßen Verfahrens bietet Vorteile in zweierlei Hinsicht:

Für die Produktabtrennung aus produkthaltigen Lösungen ist wegen der günstigen Gleichgewichtsverhältnisse eine energiesparende Produktabtrennung bei geringem apparativem Aufwand möglich.

Die Produktabtrennung in einem externen Kreislauf ermöglicht die Verarbeitung konzentrierterer Substrate bzw. Maischen, wobei der Gesamtenergieverbrauch infolge der resultierenden, viel geringeren Menge an mitzuführendem Ballastwasser drastisch reduziert wird. Gärprodukte können auf diese Weise von Zeit zu Zeit oder kontinuierlich abgetrennt werden. Zur kontinuierlichen Abtrennung kann ein Teilstrom des wässerigen Mediums aus der Fermentation abgezogen und beispielsweise in einer oder mehreren flüssig-flüssig-Extraktionskolonnen mit dem Extraktionsmittel behandelt werden. Jede Art von bekannten Extraktionskolonnen, wie Kolonnen mit Rührorganen, kann verwendet werden. Das wässerige Raffinat kann wenigstens teilweise wieder in die Fermentation rückgeführt werden.

Die Aldehyde sind im Vergleich zu den weiter oben erwähnten Extraktionsmitteln - beispielsweise höheren einwertigen Alkoholen - in wässerigen Medien bei weitem weniger löslich, weswegen die Verluste an den erfindungsgemäß eingesetzten Extraktionsmitteln auch ohne nachgeschaltete Extraktion derselben aus dem wässerigen Raffinat sehr gering gehalten werden können. Sie wirken auch weniger toxisch auf eventuell vorhandene Mikroorganismen; da sie außerdem nur mehr in äußerst geringen Konzentrationen noch im Raffinat vorhanden sind, bewirken sie praktisch keine Fermentationshemmung.

Auch die Verteilungskoeffizienten der abzutrennenden Verbindungen zwischen aldehydhältigen Extraktionsmitteln und wässerigen Medien sind höher als jene für bisher zur Extraktion eingesetzte Lösungsmittel.

Zweckmäßig wird ein Extraktionsmittel mit einem Aldehydgehalt von wenigstens 10 % eingesetzt.

Den restlichen Teil des Extraktionsmittels können andere, in Wasser schwer - bis unlösliche, mehr oder weniger polare Lösungsmittel wie höhere Alkane und Alkohole, Ketone, z.B. Diisobutylketon, Chinolin- oder Pyridinderivate bilden.

Vorzugsweise enthält das Extraktionsmittel wenigstens einen aliphatischen Aldehyd mit gerader oder verzweigter Kohlenstoffkette.

Besonders bevorzugt wird als Extraktionsmittel Pentanal, Hexanal, Heptanal oder ein Gemisch von wenigstens zweien dieser Aldehyde eingesetzt.

Die Löslichkeit von Hexanal in reinem Wasser beträgt 0,1 Massen%; Heptanal ist sogar nur zu weniger als 0,01 Massen% in reinem Wasser löslich (bezogen auf 20°C).

Die Löslichkeit der entsprechenden Alkohole in reinem Wasser ist signifikant höher, sie beträgt 0,5 Massen% für Hexanol und 0,17 Massen% für Heptanol.

Das auch bereits vielfach für Extraktionszwecke eingesetzte Diisobutylketon ist zu etwa 0,1 Massen% in wässerigen Medien löslich.

Nach einer Ausführungsform der Erfindung wird zur Abtrennung von Äthanol aus wässerigen Gär- (Fermentations-) medien Hexanal oder Heptanal als Extraktionsmittel eingesetzt.

Gemäß einer anderen Ausführungsform wird zur gleichzeitigen Abtrennung von Butanol und Aceton aus wässerigen Gär-(Fermentations-)medien Heptanal als Extraktionsmittel eingesetzt.

Die Bestimmung der Gleichgewichtsparameter erfolgte in einem auf 20°C thermostatisierten Glasdoppelmantelgefäß mit einer Abflußmöglichkeit zur Trennung der wässerigen von der organischen Phase. Das Glasdoppelmantelgefäß ermöglichte eine Beobachtung der Durchmischung und der Phasentrennung, so daß auch die Absetzgeschwindigkeit der Lösungsmittelphase von der Wasserphase abgeschätzt werden konnte. Die für den Stoffaustausch nötige innige Durchmischung erfolgte mittels eines motorbetriebenen Rührers, dessen Welle gasdicht in das Gefäß eingeführt war. So wurde die Verdampfung der abzutrennenden Verbindung, von Wasser sowie von Extraktionsmittel verhindert. Die sauber voneinander getrennten Phasen wurden in Reagenzgläser abgefüllt und letztere mit Gummistoppeln dicht verschlossen. Spätestens am darauffolgenden Tag wurden die Phasen gaschromatographisch analysiert.

Aus den Analysendaten wurden der Verteilungskoeffizient, die Selektivität und die Löslichkeit des Extraktionsmittels in Wasser ermittelt.

Die Erfindung wird in den folgenden Beispielen noch näher erläutert.

Beispiel 1:

Abtrennung von Äthanol

a) Eine wässerige Lösung mit einem Äthanolgehalt von 7 Massen% wird in dem voranstehend beschrebenen Gefäß mit der gleichen Menge an Hexanal behandelt. Man erhält eine Extraktlösung mit einem Äthanolgehalt von 3,6 Massen%. Die Äthanolkonzentration im wässerigen Raffinat sinkt auf 3,3 Massen% ab. Die Konzentration des Hexanals im Raffinat betrug nur 0,2 Massen%.

b) Unter den in a) beschriebenen Bedingungen wurde Heptanal als Extraktionsmittel eingesetzt. Die Äthanolkonzentrationen betrugen:
im Extrakt 3,2 Massen%
im Raffinat 3,8 Massen%.

Der Heptanalgehalt im wässerigen Raffinat war praktisch vernachlässigbar, was bei Anwesenheit extrem empfindlicher Mikroorganismen im wässerigen Medium von größter Bedeutung ist.

Die Verteilungskoeffizienten bei 10 % Äthanol in der Extraktphase liegen für Hexanal als Extraktionsmittel bei 1,29 und für Pentanal als Extraktionsmittel bei 1,31.

Der analog ermittelte Verteilungskoeffizient für die Verteilung von Äthanol zwischen Heptanal und Wasser betrug 0,94.

Die Extraktion von Äthanol, welche sich aufgrund der geringen Polaritätsunterschiede des Äthanols zu Wasser schwierig gestaltet, ist nach dem erfindungsgemäßen Verfahren wirtschaftlich und betriebssicher durchführbar. Die Verteilungskoeffizienten liegen vorwiegend über 1; es kann daher mit wenigen Trennstufen (d.h. mit Extraktionskolonnen geringer Bauhöhe) eine wirkungsvolle Absenkung der Produktkonzentration erreicht werden.

Vergleichsbeispiel 1:

a) Bei Einsatz von n-Hexanol als Extraktionsmittel ergibt sich eine Äthanolkonzentration im Extrakt von nur 3,3 Massen% und eine höhere Konzentration im Raffinat von 3,7 Massen%. Die Löslichkeit von Hexanol in wässerigen Medien ist größer, so daß dessen Konzentration im Raffinat 0,6 Massen% beträgt.

b) Verwendet man n-Heptanol als Extraktionsmittel, liegt die Konzentration des Äthanols im Extrakt noch tiefer, nämlich bei 3,0 Massen%. Die entsprechende Raffinatkonzentration ergibt sich mit 4,0 Massen% Äthanol.

Beispiel 2:

Abtrennung von Butanol und Aceton

a) Da z.B. bei der Aceton-Butanol-Gärung die Produktionshemmung der Mikroorganismen bereits unter 2 Massen% Butanol in der Maische (im Fermentationsmedium) beginnt, wurde bei den Extraktionsversuchen von 1,8 massen%igen wässerigen Butanollösungen ausgegangen. Die einstufige Extraktion mit gleichen Mengen Heptanal ergab 1,6 Massen% Butanol in der organischen Extraktphase und eine Verminderung der Butanol-Konzentration im wässerigen Raffinat auf 0,13 Massen%. Die Löslichkeit des Heptanals im Raffinat ist gering und liegt wie in reinem Wasser unter 0,01 %.

Der Verteilungskoeffizient für 1,7 % Butanol in der Extraktphase liegt bei 12,9.

b) Zur Anwendung des erfindungsgemäßen Verfahrens bei der Aceton-Butanol-Fermentation ist auch die gleichzeitige Abtrennung des Nebenproduktes Aceton wesentlich. Für Heptanal als Extraktionsmittel liegen die Konzentrationen, ausgehend von einem wässerigen Medium mit 2,1 Massen% Aceton, mit 0,95 Massen% im Extrakt und 1,15 Massen% im Raffinat noch deutlich besser als bei konventionellen Lösungsmitteln, wenn die Extraktion wieder einstufig wie unter a) beschrieben durchgeführt wird.

Der Verteilungskoeffizient ergibt sich mit 0,83 (Aceton zwischen Heptanal und Wasser).

Vergleichsbeispiel 2:

a) Geht man wie in Beispiel 2a) von einer Lösung mit 1,8 Massen% Butanol in Wasser aus und extrahiert einstufig mit der gleichen Menge Heptanol, befinden sich 1,65 Massen% Butanol im Extrakt und 0,15 Massen% Butanol im Raffinat. Das Raffinat enthält allerdings auch mindestens 0,1 Massen% des Extraktionsmittels.

b) Analog zu Beispiel 2b) wurden unter Einsatz von Heptanol als Extraktionsmittel folgende Konzentrationen an Aceton ermittelt:
0,8 Massen% im Extrakt
1,2 Massen% im Raffinat

Es ist ersichtlich, daß Heptanal als Extraktionsmittel zum gemeinsamen Abtrennen der Produkte Butanol, Aceton und Äthanol, welche z.B. bei der Butanol-Aceton-Gärung gleichzeitig in wässerigem Medium anfallen, besser geeignet ist als übliche Lösungsmittel.

**Patentansprüche**

1. Verfahren zur Abtrennung von sauerstoffhältigen organischen Verbindungen, wie Alkoholen, Aldehyden, Ketonen, Äthern, höheren Carbonsäuren und Estern, aus wässerigen Medien durch flüssig-flüssig-Extraktion mit einem organischen Extraktionsmittel, dadurch gekennzeichnet, daß ein Extraktionsmittel eingesetzt wird, welches einen Gehalt von bis zu 100 Massen% an wenigstens einem Aldehyd mit 4 bis 20 Kohlenstoffatomen aufweist.

2. Verfahren nach Anspruch 1, dadurch gekennzeichnet, daß ein Extraktionsmittel mit einem Aldehydgehalt von wenigstens 10 % eingesetzt wird.

3. Verfahren nach einem der Ansprüche 1 und 2, dadurch gekennzeichnet, daß das Extraktionsmittel wenigstens einen aliphatischen Aldehyd mit gerader oder verzweigter Kohlenstoffkette enthält.

4. Verfahren nach wenigstens einem der Ansprüche 1 bis 3, dadurch gekennzeichnet, daß als Extraktionsmittel Pentanal, Hexanal, Heptanal oder ein Gemisch von wenigstens zweien dieser Aldehyde eingesetzt wird.

5. Verfahren nach wenigstens einem der Ansprüche 1 bis 4, dadurch gekennzeichnet, daß zur Abtrennung von Äthanol aus wässerigen Gär-(Fermentations-)medien Hexanal oder Heptanal als Extraktionsmittel eingesetzt wird.

6. Verfahren nach wenigstens einem der Ansprüche 1 bis 4, dadurch gekennzeichnet, daß zur gleichzeitigen Abtrennung von Butanol und Aceton aus wässerigen Gär-(Fermentations-)medien Heptanal als Extraktionsmittel eingesetzt wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | PATENT ABSTRACTS OF JAPAN, Band 8, Nr. 30 (C-209)[1467], 8. Februar 1984; & JP - A - 58 193 694 (BAIORISAACHI CENTER) 11.11.1983 | 1 | B 01 D 11/04 C 12 P 7/06 C 07 C 27/34 C 07 C 29/86 C 12 P 7/16 B 01 D 11/00 |
| Y | US-A-3 939 216 (WRIGHT) * Beispiel 1 * | 1-3 | |
| Y | US-A-4 517 298 (TEDDER) * Spalte 5, Zeilen 19-32 * | 1-3 | |
| A | EP-A-0 032 445 (LEVY) * Ansprüche * | 1 | |
| A | GB-A-2 127 811 (GEBR. BECKER) * Seite 4, Zeilen 24-53 * | 1 | RECHERCHIERTE SACHGEBIETE (Int. Cl. 3) |
| A | US-A-4 508 929 (D.C. SAYLES) * Anspruch 1 * | 1 | B 01 D 11/00 C 12 P 7/00 C 07 C 27/00 C 07 C 29/00 |
| A | FR-A- 947 374 (TEXACO DEVELOPMENT CORP.) * Seite 1, Zeilen 1-44 * | 1 | |

Der vorliegende Recherchenbericht wurde für alle Patentansprüche erstellt.

| Recherchenort BERLIN | Abschlußdatum der Recherche 04-05-1987 | Prüfer PROBERT C.L. |
|---|---|---|

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82